# EUROPEAN PATENT SPECIFICATION

(11) **EP 4 117 536 B1**
(45) Date of publication and mention of the grant of the patent: **25.11.2026**
(21) Application number: 21809750.9
(22) Date of filing: 19.05.2021
(51) Int. Cl.: A61B 17/32, A61B 17/42, A61M 3/02, A61B 17/00, A61B 90/00, A61B 10/02, A61B 10/00

(54) **FLUID MANAGEMENT SYSTEMS**
FLÜSSIGKEITSVERWALTUNGSSYSTEME
SYSTÈMES DE GESTION DES LIQUIDES

(30) Priority: 20.05.2020 US 202063027554 P
(43) Date of publication of application: 18.01.2023
(73) Proprietor: Meditrina, Inc., San Jose, CA 95129 (US)
(72) Inventor: TRUCKAI, Csaba, Saratoga, CA 95070 (US); TRUCKAI, Tamas J., Saratoga, CA 95070 (US)
(74) Representative: Lorenz Seidler Gossel Part. mbB
(86) International application number: PCT/US2021/070583
(87) International publication number: WO 2021/237242

(56) References cited:
- WO-A1-2016/028507
- WO-A1-2019/189078
- WO-A1-2020/097448
- US-A1- 2011 125 055
- US-A1- 2017 360 466
- US-A1- 2017 360 466
- US-A1- 2018 250 029
- US-A1- 2019 104 932
- US-B2- 9 655 639
- US-B2- 9 655 639

## Description

### BACKGROUND OF THE INVENTION

Field of the Invention. The present invention relates to single-use devices that carry at least one peristaltic pump for providing fluid flows in hysteroscopic or similar procedures that in one variation includes a single-use handheld device with a motor-driven cutter and an integrated peristaltic pump that is used for resecting and removing tissue in an endoscopic procedure.

Prior art tissue resecting devices are shown, e.g., by US 2017/360466 A1, US 9 655 639 B2 and WO 2020/097448 A1, wherein US 2017/360466 shows a tissue resecting device comprising a handle carrying a motor drive, an elongated sleeve assembly coupled to the handle and including an outer sleeve and an inner sleeve, a peristaltic pump and furthermore a tissue trap carried by the handle.

### SUMMARY OF THE INVENTION

The tissue resecting devices according to the invention are defined by claim 1, wherein preferred embodiments are laid down in the dependent claims. The tissue resecting device comprises a handle carrying a motor drive. The tissue resecting device also comprises an elongated sleeve assembly coupled to the handle. The elongated sleeve has an outer sleeve with a distal opening for receiving tissue. The elongated sleeve also has a moveable inner sleeve adapted to resect tissue received by a window at a distal end of the inner sleeve. The tissue resecting device also comprises a peristaltic pump and a tissue trap carried by the handle.

The motor drive is adapted to move the inner sleeve and resect tissue. The motor drive is also adapted to provide negative pressure in a passageway in the inner sleeve to thereby aspirate fluid and resected tissue through the passageway and into the tissue trap. The motor drive can move the inner sleeve axially, rotationally, or both. The tissue trap can be positioned proximally or distally relative to the motor drive. The peristaltic pump can have a rotational axis aligned with the rotational axis of a shaft of the motor drive. The tissue trap can be detachable from the handle. The tissue trap can include a transparent material.

The motor drive can further be adapted to operate at a plurality of selected speeds to thereby provide a corresponding plurality of selected levels of negative pressure. The motor drive can be adapted to rotate the inner sleeve at a selected speed between 100 rpm and 5,000 rpm. The motor drive can also be adapted to adapted provide a negative pressure to cause an outflow rate between 10 ml/min and 1,000 ml/min.

The tissue resecting device can further comprise a gear mechanism. The gear mechanism can provide oscillating rotation of the inner sleeve with the motor drive rotating in a single direction. The tissue resecting device can further comprise a second peristaltic pump carried by the handle. The second peristaltic pump can be adapted for providing fluid inflows from a fluid source through a channel in the sleeve assembly to a distal end thereof.

The present disclosure also includes fluid management systems. For example, a variation of such a fluid management can comprise a housing, a first peristaltic pump, a second peristaltic pump, and at least one motor. The first peristaltic pump can be carried by the housing and can be adapted to provide fluid inflow to a treatment site. The first peristaltic can engage flexible tubing that extends from a fluid source. The second peristaltic pump can be carried by the housing and can be adapted to provide fluid outflows from the treatment site. The second peristaltic pump can engage flexible tubing that extends to a tissue catch. The at least one motor can be carried by the housing and can operate the first and second peristaltic pumps.

The at least one motor can be adapted to provide inflow and outflow rates between 10 ml/min and 1,000 ml/min. The single-use fluid management system can further comprise a pressure sensor adapted to measure fluid pressure in the fluid inflow. The single-use fluid management system can further comprise a controller adapted to maintain a set pressure in a working space responsive to signals from the pressure sensor.

The descriptions provided herein are examples of the invention described herein and defined by the claims. It is contemplated that combinations of specific embodiments, specific aspects or combinations of the specific embodiments themselves are within the scope of this disclosure.

### BRIEF DESCRIPTION OF THE DRAWINGS

Additional aspects of the invention will become clear from the following description of illustrative embodiments and from the attached drawings, in which:
FIG. 1 is a perspective view of single-use tissue resecting device which includes a handle carrying a motor drive adapted to rotate a tubular cutter at a distal working end of an elongate shaft assembly wherein the motor drive is adapted to contemporaneously operate a peristaltic pump within the handle that is adapted to suction fluid and resected tissue from a treatment site.
FIG. 2 is a perspective view of the device of FIGS. 1 and 2 showing the handle partly disassembled to show the peristaltic pump, the motor drive and a tissue catch together with an enlarged view of the working end of the elongate shaft assembly.
FIG. 3 is an exploded view of the components in the interior of the handle of FIG. 1, showing the peristaltic pump, the motor drive and the tissue catch.
FIG. 4 is an exploded view of another variation of a device similar to that of FIGS. 1-3, wherein the components in the interior of the handle include an inflow peristaltic pump, an outflow peristaltic pump, a motor drive, a tissue catch and a gear assembly configured for converting unidirectional rotation of the motor shaft into an oscillating rotational movement of a tubular cutting member in the working end of the shaft assembly.
FIG. 5 is an enlarged perspective view of the working end of the device of FIG. 4, which is similar to the working end of FIG. 2, showing the fluid inflow channel communicating with the inflow peristaltic pump and further showing the tissue extraction passageway communicating with the outflow peristaltic pump.
FIG. 6 is an exploded view of a single-use fluid management system wherein a housing carries an inflow peristaltic pump, an outflow peristaltic pump, a motor drive and a tissue catch.
FIG. 7 is a schematic view of the single-use fluid management system of FIG. 6.
FIG. 8 is another view of the single-use fluid management system of FIGS. 6 and 7 showing the system components mounted on a roll-stand.

### DETAILED DESCRIPTION OF THE INVENTION

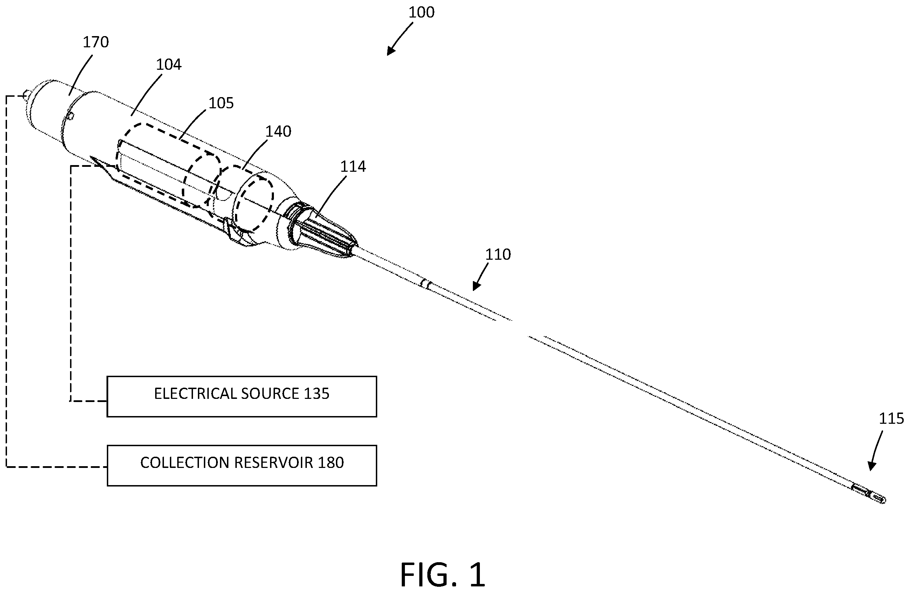

FIGS. 1 through 3 illustrate a motor-driven resecting device 100 that is designed for single-use, typically in a hysteroscopic procedure to resect and remove a polyp, a fibroid or other abnormal uterine tissue. As is known in the art, sterilization of re-usable handles with motor drives is complex and expensive. The cost of sterilization as well as the risks of infection from improperly sterilized devices makes single-use device less expensive to a hospital or physician's office, when compared to using sterilizable devices.

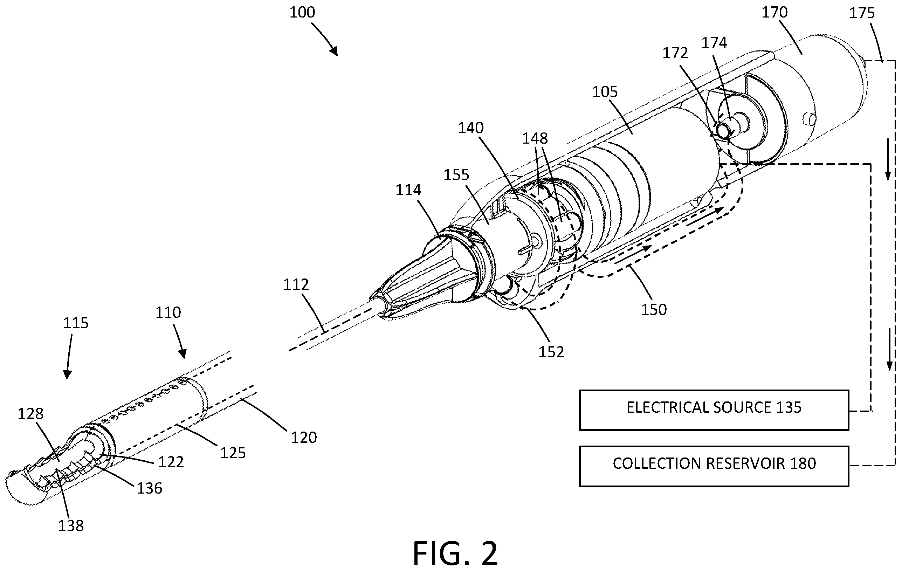

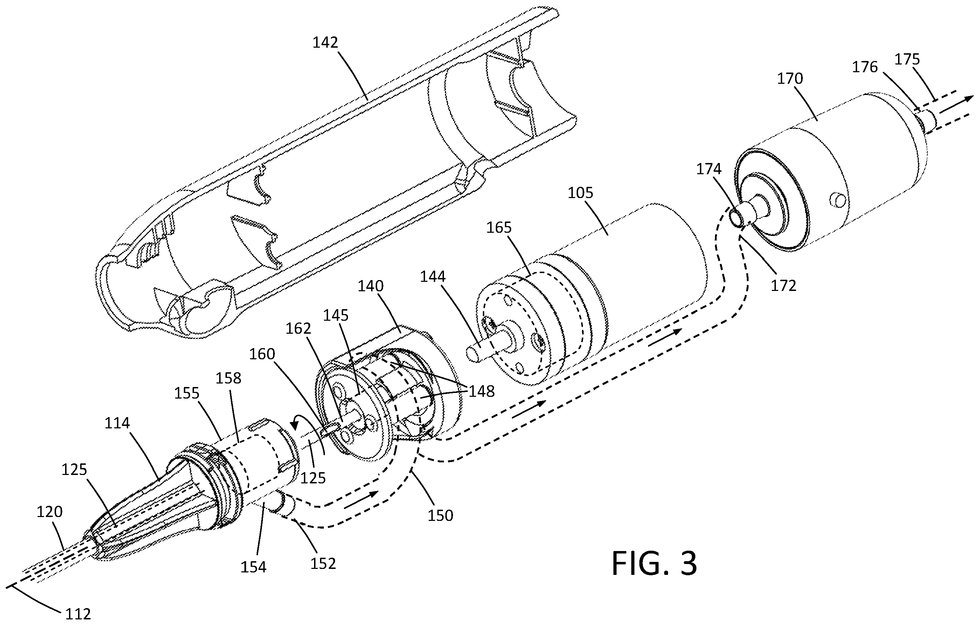

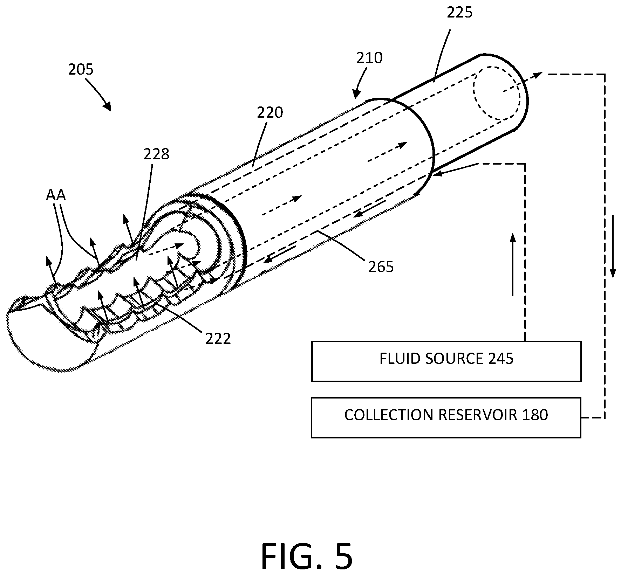

Referring to FIGS. 1 and 2, the handle or handpiece 104 includes a motor drive 105 and is coupled to an elongate shaft or sleeve assembly 110 extending about longitudinal axis 112. The shaft assembly 110 extends distally from a rotatable hub 114 to the working end 115. In one variation, the elongated shaft assembly 110 comprises an outer sleeve 120 with a distal window 122 and a motor-driven, rotating inner sleeve 125 with a window 128 therein (see FIGS. 2 and 5). The motor drive 105 is coupled to an electrical source 135 and is adapted to rotate the inner sleeve 125 as further described below. (see FIGS. 2 and 3). Such a type of tubular cutter is known in the art wherein the rotating inner sleeve 125 cuts tissue that interfaces with window 122 in the outer sleeve 120 as the inner sleeve window 128 rotates or oscillates at high speed. Either or both windows 122 and 128 may be configured with cutting teeth, with FIG. 2 illustrating teeth 136 on outer window 122 and teeth 138 on inner window 128.

As can be understood from FIGS. 1 and 2, the rotating hub 114 of the shaft assembly 110 is coupled to the handle 104 so that the physician can rotate the shaft assembly 110 and working end 115 relative to the handle 104 to any rotational orientation for cutting tissue while maintaining the handle 104 in an upright or stable position. The handle 104 can include an actuator button (not shown) for actuating the motor drive 105 or a footswitch can be used.

Referring to FIG. 1 and 2, in one variation, the tissue resecting device 100 has a shaft assembly 110 with a diameter ranging from 2 mm to 6 mm, and more often the diameter is between 3 mm and 5 mm. The shaft assembly 110 has a diameter and length for cooperating with a working channel of an endoscopic viewing system or other introducer.

As is known in the prior art, typically a fluid management system is used in a hysteroscopic resection procedures to expand a patient's uterine cavity to allow for endoscopic viewing. In one variation shown in FIGS. 1 and 2, the resecting device 100 includes a peristaltic pump 140 carried in the interior of the handle 104 which thus comprises a component of a fluid management system. More in particular, the peristaltic pump 140 is configured to provide fluid outflows from a treatment site, where the fluid inflows can be provided by a simple fluid inflow mechanism, such as gravity flow from a hanging saline bag.

FIG. 2 shows the handle 104 of FIG. 1 with a first side of the exterior shell 142 removed to show the motor drive 105 and peristaltic pump 140. In a variation, the handle 104 carries an inexpensive, DC electric motor drive 105 which allows for its disposability. As can be seen in the exploded view of FIG. 3, the motor drive 105 has a drive shaft 144 that connects a central shaft 145 of the peristaltic pump 140 which has rollers 148 aligned with the axis 112 of the drive shaft 144. The peristaltic pump 140 is of a conventional design with 3 or 4 rollers that are adapted to engage flexible tubing 150 shown in phantom view. The flexible tubing 150 has a distal end 152 that couples to a fitting 154 in a housing portion 155 of the rotatable hub 114. The flexible tubing 150 can be disposed with a space in the handle 104 or can be secured by retention clips in an inferior surface of the handle. The interior lumen of the tubing 150 communicates with a chamber 158 in the housing 155 which is open to a fluid port 160 in the proximal end 162 of the rotating inner sleeve 125 (FIG. 3). In FIG. 3, it further can be seen that the proximal end 162 of inner sleeve 125 is fixed to the central shaft 145 of the peristaltic pump 140. Thus, in the variation of FIGS. 2 and 3, the motor drive 105 is configured to rotate the inner sleeve 125 and peristaltic pump 140 at the same RPM, which can be from 100 RPM to 5,000 RPM. In one variation, the motor drive 105 provides a negative pressure to cause an outflow rate between 10 ml/min and 1,000 ml/min.

Referring again to FIGS. 2 and 3, the handle 104 further carries a tissue trap 170 of a type know in the art where fluid outflow and tissue chips are carried into the tissue trap 170 by the peristaltic pump 140 and the tissue trap has a filter that captures the resected tissue chips in a filter, where the tissue chips can be collected for biopsy purposes. In one variation, the tissue trap 170 is made of a transparent material to allow viewing of the tissue chips. In FIG. 3, the direction of fluid outflows is indicated by arrows in the tubing 150. The proximal end 172 of the tubing is connected to a fitting 174 in the tissue trap 170. Another length of tubing 175 couples to a proximal fitting 176 of the tissue trap 170 which extends to a remote collection reservoir 180 (FIGS. 1-2).

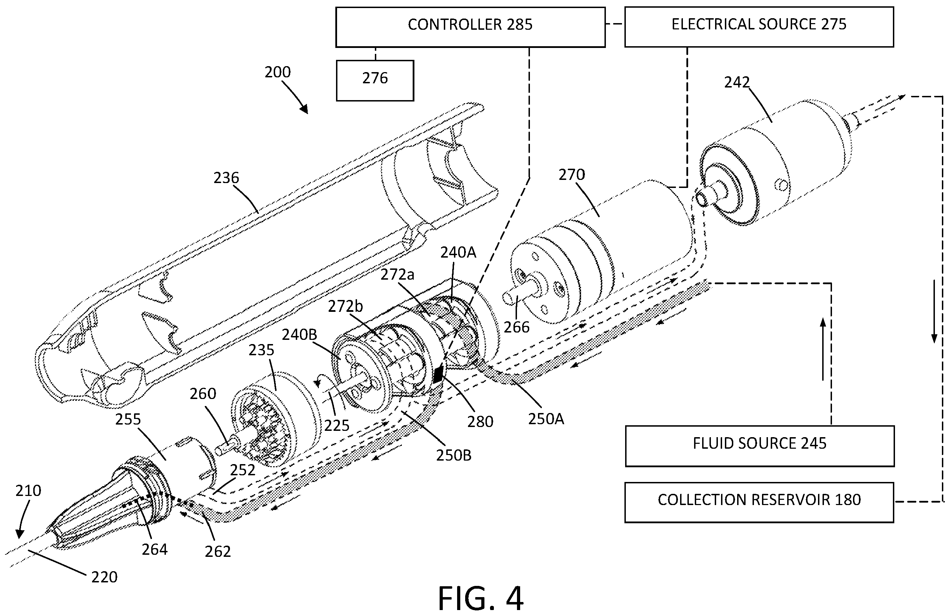

FIGS. 4 is an exploded view of another variation of a resection device 200 and FIG. 5 is an enlarged view of the working end 205 of the device 200 of FIG. 4. In this variation, the shaft assembly 210 remains the same with an outer sleeve 220 and window 222 with a motor-driven inner sleeve 225 and window 228 for resecting tissue.

The resection device 200 of FIGS. 4 and 5 differs from the previous embodiment in that a gear mechanism 235 is carried within the handle 236 to oscillate rotation of the inner sleeve 225 instead of unidirectional rotation. Further, the handle 236 carries a first peristaltic pump 240A for providing fluid inflows and a second peristaltic pump 240B for providing fluid outflows as described previously. In one aspect of the invention, the device 200 carries all the components of a fluid management system with both inflow and outflow pumps and a tissue catch 242. In the exploded view of FIG. 4, the first inflow peristaltic pump 240A is in fluid communication with a fluid source 245, such as a saline bag. The pump 240A engages flexible tubing 250A that extends to the fluid source 245. The outflow peristaltic pump 240B engages flexible tubing 250B which operates as described previously, with the distal end 252 of the outflow tubing 250B communicating with a chamber (nor shown) in housing 255 which receives fluid and tissue chips from port 260 in inner sleeve 225 (see FIG. 3).

As can be understood from FIGS. 4 and 5, the distal end 262 of inflow tubing 250 a connect to a passageway 264 in housing 255 that further communicates with in an inflow channel 265 in the shaft assembly 210 which consists of the annular space in the bore of the outer sleeve 220 and outward of the outer surface of the inner sleeve 225 (FIG. 5). Turning to FIG. 5 which shows the working end 205, it can be seen that fluid inflows are indicated by arrows AA where the fluid exits the space between the inner sleeve 225 and outer sleeve 220. The motor drive 270 can be adapted provide inflow and outflow rates between 10 ml/min and 1,000 ml/min. The motor drive 270 is operatively coupled to an electrical source 275 which may be a battery in the handle or a remote battery or power source. Actuation of the system can be provided by a switch 276 in the handle or a foot switch.

Referring to FIG. 4, both peristaltic pumps 240A and 240B can be a conventional design with 3 or 4 rollers engage the flexible tubing 250A and 250B, respectively. Both pumps 240A and 240B can be connected to motor shaft 266 of motor drive 270. As can be seen in FIG. 4, the pumps 240A and 240B can rotate in the same direction, but the flexible tubing 250A and 250B can be disposed around the roller 272a and 272b of the pumps in opposing directions (i.e., one clockwise and the other counterclockwise) to that fluid flow in opposing directions in the tubing. In other variations, the handle 232 can be provided with a single motor and gear mechanisms to drive the pumps in opposing directions, or the handle can be provided with an individual motor for each pump. While peristaltic pumps 240A and 240B are shown in the figures, it should be appreciated that other types of pumps can be used, such as piston pumps, impeller pumps, vane pumps and the like.

Referring again to FIG. 4, the exploded view of handle 236 shows the gear mechanism 235 which converts the single direction of rotation of the motor drive shaft 266 to an oscillating rotation of the inner sleeve 225 to thus provide and oscillating movement of the inner sleeve cutting window 228 at the working end 205 of the device 200 (See FIG. 5). As is well known in the field of tissue resection, tubular cutters work optimally when the inner rotating sleeve and inner cutting window 228 oscillates, for example, with several revolutions in one rotational direction followed by a similar number of rotations in the opposite direction. Such oscillation provides improved cutting performance when compared with devices that rotate a cutting member in a single direction. The gear mechanism 235 is more fully described in commonly owned U.S. Patent Application Number 16/678,647 titled ENDOSCOPE AND METHOD OF USE filed November 8, 2019. The gear mechanism 235 can operate at any suitable rotation speed, for example 100 RPM to 5000 RPM or more.

In another aspect of the invention, the peristaltic pumps 240A and 240B of the resecting device 200 can provide different flow rates, wherein one flow rate (inflow or outflow rate) can range from 50% to 100% of the other flow rate. Such varied inflow and outflow rates can be provided for any constant pump rotational speed by varying the interior lumen diameter of the inflow tubing 250A and the outflow tubing 250B. Alternatively, a gear mechanism can be provided to rotate the pumps at different rotational speeds, for any given motor speed.

In another optional variation, a device 200 as in FIG. 4 can include a pressure sensor 280 positioned to measure pressure in the inflow line 250A distal to the pump 250A. The pressure sensor 280 can be configured to send pressure signals to a processor or controller 285 which in turn can include control algorithms for actuation of the pumps. In another variation, the device 200 can be provided with first and second motor drives (not shown) coupled to the respective pumps 240A and 240B, and the controller 285 can control operation of the pumps independently to maintain a set pressure in a treatment site based on pressure readings by the pressure sensor 280.

While FIG. 4 shows two separate peristaltic pumps 240A and 240B, it should be appreciated that a single peristaltic pump with elongated rollers may be configured with inflow and outflow tubing disposed opposing rotational directions around the roller to provide the inflows and outflows.

While FIG. 4 shows on variation of a handle design in which the pumps 240A and 240B, gear mechanism 235, motor 270 and tissue catch 242 are positioned in a particular longitudinal arrangement, these components can be positioned in any suitable manner within the handle 236.

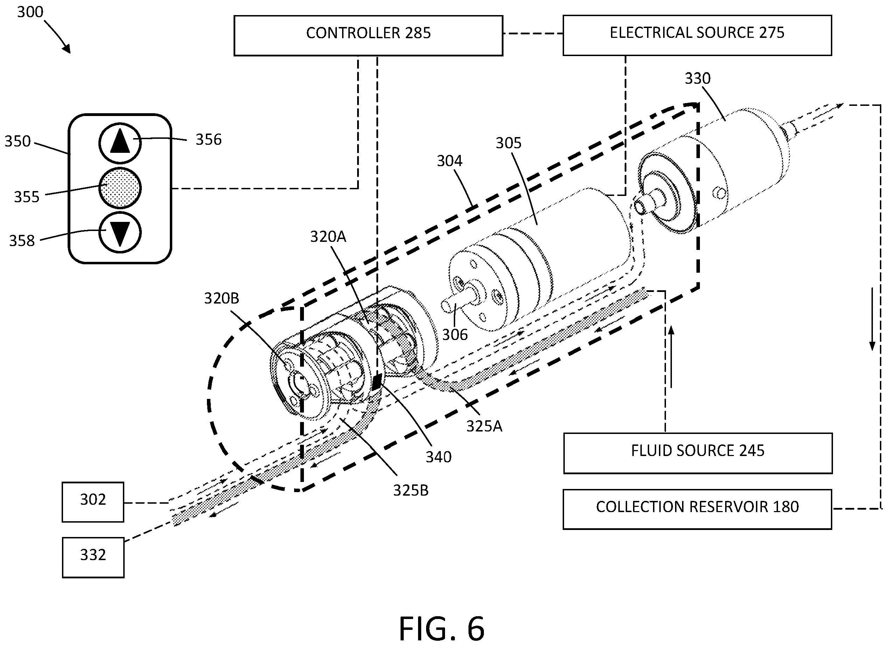

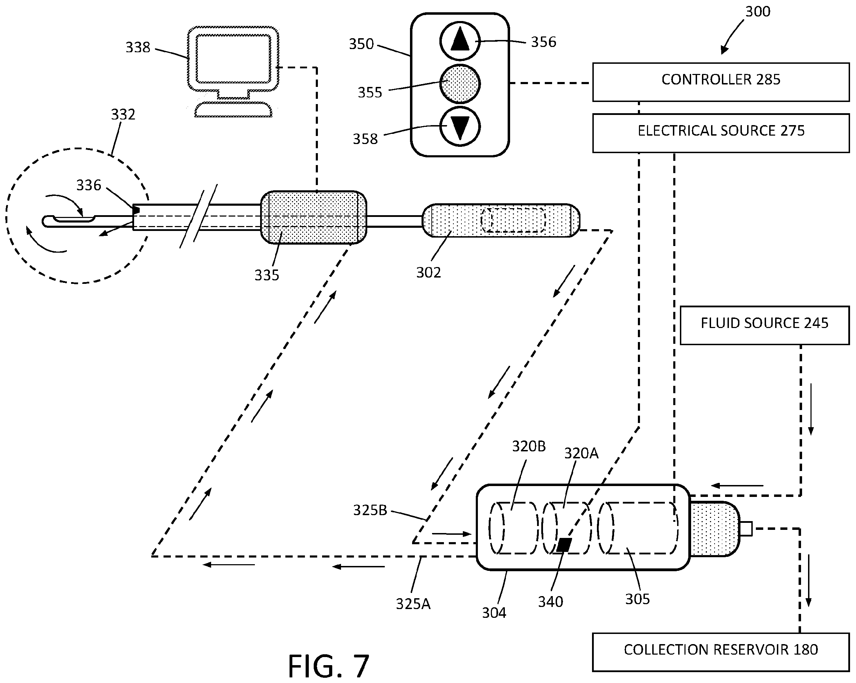

FIGS. 6, 7 and 8 illustrate schematically another variation of the invention which comprises a single-use fluid management system 300 that can be used for diagnostic procedures or that can be used with a separate resection device 302 in shown in FIG. 7 in a resection procedure. In a hysteroscopic diagnostic procedure, both the fluid inflow and outflow lines would be connected to an endoscope. In a resection procedure, the fluid inflow would be connected to an endoscope and the outflow line would be connected to the resection device.

**In** FIG, 6, it can be seen that the fluid management system 300 consists of a housing 304 that carries a motor drive 305 with motor shaft 306 that drives peristaltic pump portions 320A and 320B that are similar to the pumps in the previously described system. The peristaltic pumps 320A and 320B engage respective tubing sets 325A and 325B to provide fluid inflows and outflows. A fluid source 245 and collection reservoir 180 are provided as in the previous system. A tissue catch 330 also is the same as described previously. The fluid inflows from pump 320A are configured to flow into a working space 332, such as a uterine cavity, as shown in FIG. 7. The inflow can be directed through an inflow channel in an electronic endoscope 335 with image sensor 336 as shown in FIG. 7. The endoscope 335 is coupled to display 338. The motor drive 305 is connected to an electrical source 275 which is either a battery in the housing 304 or a remote battery or power source, which may be connected to the controller 285 further described below.

**In** the fluid management system 300 of FIGS. 6 and 7, a pressure sensor 340 again is positioned to measure pressure in the inflow line 325A distal to the pump 320A. The pressure sensor 340 is configured to send pressure signals to the processor or controller 285. In this variation, the controller 285 may be adapted for single use or can be re-useable and carried in suitable housing which also may carry the battery or power source 275. The controller 285 includes control algorithms for actuation of the pumps 320A and 320B for maintaining a set pressure in the working space 332, such as a uterine cavity, in response to pressure signals from the sensor 340.

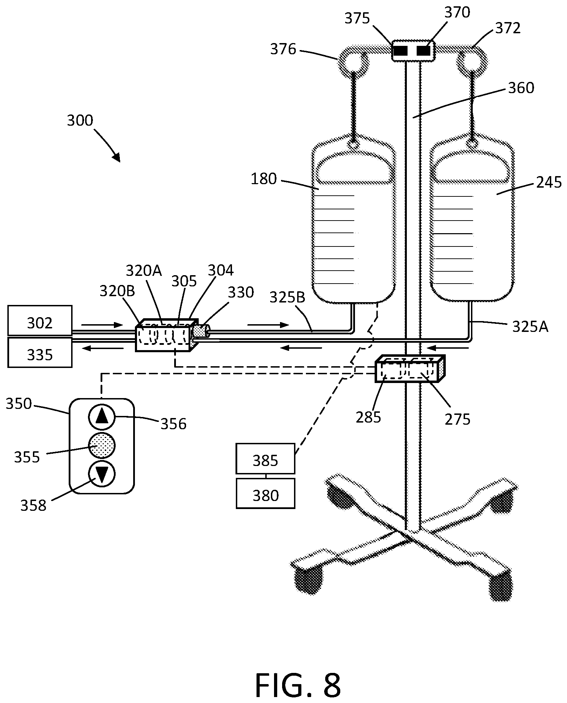

**In** order to operate the fluid management system 300, a control pad 350 is coupled to the controller 285, which can include an ON/OFF button 355 for actuating the pumps. Further, the control pad 350 can include buttons 356 and 358 for increasing and decreasing the set pressure. Another button (not shown) can be provided for a "flush" mode wherein the fluid flow rate is increased to a higher level for flushing the working space 332. The control pad can be disposable and attached to the handle of the endoscope 335 when performing a diagnostic procedure or can be attached to the handle of a resecting device 302 in a resection procedure. In use, a saline bag comprising the fluid source 245 and the collection reservoir (e.g., a plastic bag) 180 can be hung on a stand 360 as shown in FIG. 8. The controller 285 can comprise a small housing that also can be attached to the stand 360 carrying the saline bag 245. The single-use disposable fluid management system 300 can be positioned in any suitable location for coupling to the endoscope 335 and optionally to the resecting device 302. The system thus occupies a very small footprint compared to commercially available fluid management systems.

**In** a variation shown in FIG. 8, the stand 360 can include a first load sensor 370 operatively connected to a first hook 372 carrying the saline bag 245 (fluid source) and a second load sensor 375 connected to a second hook 376 carrying the collection reservoir 180, wherein both load sensors send signals to the controller 285. The controller 285 then can use controller algorithms as known in the art to calculate a fluid deficit. Other variations can include using a single load sensor that weighs both the saline bag 245 and the collection reservoir 180 as is known in the art. In another variation, the system can include a drape 380 and separate pump 385 for pumping fluid collected by a drape 380 to the collection reservoir 180 which can allow for more accurate calculation of fluid deficits.

Although particular embodiments of the present invention have been described above in detail, it will be understood that this description is merely for purposes of illustration and the above description of the invention is not exhaustive. Specific features of the invention are shown in some drawings and not in others, and this is for convenience only and any feature may be combined with another in accordance with the invention.

Certain illustrated embodiments are shown in the drawings and have been described above in detail. It should be understood, however, that there is no intention to limit the invention to the specific form or forms disclosed, but on the contrary, the intention is to cover all modifications falling within the scope of the invention, as defined in the appended claims.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. The term "connected" is to be construed as partly or wholly contained within, attached to, or joined together, even if there is something intervening. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate embodiments of the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. A tissue resecting device comprising:
a handle (104; 236) carrying a motor drive (105; 270); comprising a drive shaft (144)
an elongated sleeve assembly (110) coupled to the handle (104; 236), the elongated sleeve (120) having an outer sleeve (120; 220) with a distal opening for receiving tissue and a moveable inner sleeve (125; 225) adapted to resect tissue received by an inner window (128; 228) at a distal end of the inner sleeve, wherein the inner window (128; 228) comprises a plurality of inner teeth (138) configured to resect tissue received in the distal opening;
a peristaltic pump (140; 240A, 240B) carried by the handle (104; 236), wherein the peristaltic pump comprises rollers (148), wherein a proximal end (162) of the inner sleeve (125) is fixed to a central shaft (145) of the peristaltic pump (140), wherein the rollers (148) are aligned with an axis (112) of the drive shaft (144); and
a tissue trap (170) carried by the handle (104; 236);
wherein the motor drive (105; 270) is adapted to move the inner sleeve (125; 225) to resect tissue, and adapted to actuate the peristaltic pump (140; 240A, 240B) to provide negative pressure in a passageway in the inner sleeve (125; 225) to aspirate fluid and resected tissue through the passageway and into the tissue trap (170).

2. The tissue resecting device of claim 1 wherein the motor drive (105; 270) moves the inner sleeve (125; 225) rotationally.

3. The tissue resecting device of claim 1 wherein the motor drive (105; 270) moves the inner sleeve (125; 225) axially.

4. The tissue resecting device of claim 1 wherein the motor drive (105; 270) moves the inner sleeve (125; 225) axially and rotationally.

5. The tissue resecting device of claim 1 wherein the tissue trap (170) is positioned proximally relative to the motor drive (105; 270).

6. The tissue resecting device of claim 1 wherein the peristaltic pump (140; 240A, 240B) is positioned distally relative to the motor drive (105; 270).

7. The tissue resecting device of claim 1 wherein the peristaltic pump (140; 240A, 240B) has a rotational axis aligned with the rotational axis of a shaft of the motor drive (105; 270).

8. The tissue resecting device of claim 1 wherein the tissue trap (170) is detachable from the handle (104; 236).

9. The tissue resecting device of claim 1 wherein the tissue trap (170) includes a transparent material.

10. The tissue resecting device of claim 1 wherein the motor drive (105; 270) is adapted to operate at a plurality of selected speeds to thereby provide a corresponding plurality of selected levels of negative pressure.

11. The tissue resecting device of claim 1 wherein the motor drive (105; 270) is adapted to rotate the inner sleeve (125; 225) at a selected speed between 100 rpm and 5,000 rpm.

12. The tissue resecting device of claim 1 wherein the motor drive (105; 270) is adapted to provide a negative pressure to cause an outflow rate between 10 ml/min and 1,000 ml/min.

13. The tissue resecting device of claim 1 further comprising a gear mechanism for oscillating rotation of the inner sleeve (125; 225) with the motor drive (105; 270) rotating in a single direction.

14. The tissue resecting device of claim 1 further comprising a second peristaltic pump (240B) carried by the handle (104; 236) adapted for providing fluid inflows from a fluid source through a channel in the sleeve assembly (110) to a distal end thereof.

15. The tissue resecting device of claim 14 further comprising a pressure sensor adapted to measure fluid pressure of the fluid inflow and a controller adapted to maintain a set pressure in a working space responsive to signals from the pressure sensor.

## Patentansprüche

1. Geweberesektionsvorrichtung, umfassend:
einen Handgriff (104; 236), der einen Motorantrieb (105; 270) trägt; umfassend eine Antriebswelle (144)
eine längliche Hülsenanordnung (110), die mit dem Handgriff (104; 236) gekoppelt ist, wobei die längliche Hülse (120) eine äußere Hülse (120; 220) mit einer distalen Öffnung zur Aufnahme von Gewebe und eine bewegliche innere Hülse (125; 225) aufweist, die dazu ausgebildet ist, Gewebe zu resezieren, das von einem inneren Fenster (128; 228) an einem distalen Ende der inneren Hülse aufgenommen wird, wobei das innere Fenster (128; 228) eine Vielzahl von inneren Zähnen (138) umfasst, die dazu ausgebildet sind, in der distalen Öffnung aufgenommenes Gewebe zu resezieren;
eine Peristaltikpumpe (140; 240A, 240B), die von dem Handgriff (104; 236) getragen wird, wobei die Peristaltikpumpe Rollen (148) umfasst, wobei ein proximales Ende (162) der inneren Hülse (125) an einer zentralen Welle (145) der Peristaltikpumpe (140) befestigt ist, wobei die Rollen (148) mit einer Achse (112) der Antriebswelle (144) ausgerichtet sind; und
eine Gewebefalle (170), die von dem Handgriff (104; 236) getragen wird;
wobei der Motorantrieb (105; 270) dazu ausgebildet ist, die innere Hülse (125; 225) zu bewegen, um Gewebe zu resezieren, und dazu ausgebildet ist, die Peristaltikpumpe (140; 240A, 240B) zu betätigen, um einen Unterdruck in einem Durchgang in der inneren Hülse (125; 225) bereitzustellen, um Fluid und reseziertes Gewebe durch den Durchgang und in die Gewebefalle (170) zu aspirieren.

2. Geweberesektionsvorrichtung nach Anspruch 1, wobei der Motorantrieb (105; 270) die innere Hülse (125; 225) drehbar bewegt.

3. Geweberesektionsvorrichtung nach Anspruch 1, wobei der Motorantrieb (105; 270) die innere Hülse (125; 225) axial bewegt.

4. Geweberesektionsvorrichtung nach Anspruch 1, wobei der Motorantrieb (105; 270) die innere Hülse (125; 225) axial und drehbar bewegt.

5. Geweberesektionsvorrichtung nach Anspruch 1, wobei die Gewebefalle (170) proximal relativ zum Motorantrieb (105; 270) positioniert ist.

6. Geweberesektionsvorrichtung nach Anspruch 1, wobei die Peristaltikpumpe (140; 240A, 240B) distal relativ zum Motorantrieb (105; 270) positioniert ist.

7. Geweberesektionsvorrichtung nach Anspruch 1, wobei die Peristaltikpumpe (140; 240A, 240B) eine Drehachse aufweist, die mit der Drehachse einer Welle des Motorantriebs (105; 270) ausgerichtet ist.

8. Geweberesektionsvorrichtung nach Anspruch 1, wobei die Gewebefalle (170) vom Handgriff (104; 236) lösbar ist.

9. Geweberesektionsvorrichtung nach Anspruch 1, wobei die Gewebefalle (170) ein transparentes Material beinhaltet.

10. Geweberesektionsvorrichtung nach Anspruch 1, wobei der Motorantrieb (105; 270) dazu ausgebildet ist, mit einer Vielzahl von gewählten Geschwindigkeiten zu arbeiten, um dadurch eine entsprechende Vielzahl von gewählten Niveaus von Unterdruck bereitzustellen.

11. Geweberesektionsvorrichtung nach Anspruch 1, wobei der Motorantrieb (105; 270) dazu ausgebildet ist, die innere Hülse (125; 225) mit einer gewählten Geschwindigkeit zwischen 100 U/min und 5.000 U/min zu drehen.

12. Geweberesektionsvorrichtung nach Anspruch 1, wobei der Motorantrieb (105; 270) dazu ausgebildet ist, einen Unterdruck bereitzustellen, um eine Ausflussrate zwischen 10 ml/min und 1.000 ml/min zu bewirken.

13. Geweberesektionsvorrichtung nach Anspruch 1, ferner umfassend einen Getriebemechanismus für eine oszillierende Drehung der inneren Hülse (125; 225), wobei sich der Motorantrieb (105; 270) in einer einzigen Richtung dreht.

14. Geweberesektionsvorrichtung nach Anspruch 1, ferner umfassend eine zweite Peristaltikpumpe (240B), die vom Handgriff (104; 236) getragen wird und dazu ausgebildet ist, Fluidzuflüsse von einer Fluidquelle durch einen Kanal in der Hülsenanordnung (110) zu einem distalen Ende davon bereitzustellen.

15. Geweberesektionsvorrichtung nach Anspruch 14, ferner umfassend einen Drucksensor, der dazu ausgebildet ist, den Fluiddruck des Fluidzuflusses zu messen, und eine Steuerung, die dazu ausgebildet ist, ansprechend auf Signale vom Drucksensor einen Solldruck in einem Arbeitsraum aufrechtzuerhalten.

## Revendications

1. Dispositif de résection de tissu comprenant :
une poignée (104 ; 236) portant un entraînement à moteur (105 ; 270) ;
comprenant un arbre d'entraînement (144)
un ensemble de manchon allongé (110) couplé à la poignée (104 ; 236), le manchon allongé (120) ayant un manchon extérieur (120 ; 220) avec une ouverture distale pour recevoir du tissu et un manchon intérieur mobile (125 ; 225) apte à réséquer du tissu reçu par une fenêtre intérieure (128 ; 228) à une extrémité distale du manchon intérieur, dans laquelle la fenêtre intérieure (128 ; 228) comprend une pluralité de dents intérieures (138) configurées pour réséquer du tissu reçu dans l'ouverture distale ;
une pompe péristaltique (140 ; 240A, 240B) portée par la poignée (104 ; 236),
dans laquelle la pompe péristaltique comprend des rouleaux (148), dans laquelle une extrémité proximale (162) du manchon intérieur (125) est fixée à un arbre central (145) de la pompe péristaltique (140), dans laquelle les rouleaux (148) sont alignés sur un axe (112) de l'arbre d'entraînement (144) ; et
un piège à tissu (170) porté par la poignée (104 ; 236) ;
dans lequel l'entraînement à moteur (105 ; 270) est apte à déplacer le manchon intérieur (125 ; 225) pour réséquer du tissu, et apte à actionner la pompe péristaltique (140 ; 240A, 240B) pour fournir une pression négative dans un passage dans le manchon intérieur (125 ; 225) pour aspirer du fluide et du tissu réséqué à travers le passage et dans le piège à tissu (170).

2. Dispositif de résection de tissu selon la revendication 1, dans lequel l'entraînement à moteur (105 ; 270) déplace le manchon intérieur (125 ; 225) en rotation.

3. Dispositif de résection de tissu selon la revendication 1, dans lequel l'entraînement à moteur (105 ; 270) déplace le manchon intérieur (125 ; 225) axialement.

4. Dispositif de résection de tissu selon la revendication 1, dans lequel l'entraînement à moteur (105 ; 270) déplace le manchon intérieur (125 ; 225) axialement et en rotation.

5. Dispositif de résection de tissu selon la revendication 1, dans lequel le piège à tissu (170) est positionné de manière proximale par rapport à l'entraînement à moteur (105 ; 270).

6. Dispositif de résection de tissu selon la revendication 1, dans lequel la pompe péristaltique (140 ; 240A, 240B) est positionnée de manière distale par rapport à l'entraînement à moteur (105 ; 270).

7. Dispositif de résection de tissu selon la revendication 1, dans lequel la pompe péristaltique (140 ; 240A, 240B) a un axe de rotation aligné sur l'axe de rotation d'un arbre de l'entraînement à moteur (105 ; 270).

8. Dispositif de résection de tissu selon la revendication 1, dans lequel le piège à tissu (170) est détachable de la poignée (104 ; 236).

9. Dispositif de résection de tissu selon la revendication 1, dans lequel le piège à tissu (170) inclut un matériau transparent.

10. Dispositif de résection de tissu selon la revendication 1, dans lequel l'entraînement à moteur (105 ; 270) est apte à fonctionner à une pluralité de vitesses sélectionnées pour fournir ainsi une pluralité correspondante de niveaux sélectionnés de pression négative.

11. Dispositif de résection de tissu selon la revendication 1, dans lequel l'entraînement à moteur (105 ; 270) est apte à faire tourner le manchon intérieur (125 ; 225) à une vitesse sélectionnée entre 100 tr/min et 5 000 tr/min.

12. Dispositif de résection de tissu selon la revendication 1, dans lequel l'entraînement à moteur (105 ; 270) est apte à fournir une pression négative pour provoquer un débit de sortie entre 10 ml/min et 1 000 ml/min.

13. Dispositif de résection de tissu selon la revendication 1, comprenant en outre un mécanisme d'engrenage pour une rotation oscillante du manchon intérieur (125 ; 225), l'entraînement à moteur (105 ; 270) tournant dans une seule direction.

14. Dispositif de résection de tissu selon la revendication 1, comprenant en outre une seconde pompe péristaltique (240B) portée par la poignée (104 ; 236) apte à fournir des afflux de fluide depuis une source de fluide à travers un canal dans l'ensemble de manchon (110) jusqu'à une extrémité distale de celui-ci.

15. Dispositif de résection de tissu selon la revendication 14, comprenant en outre un capteur de pression apte à mesurer la pression de fluide de l'afflux de fluide et une unité de commande apte à maintenir une pression de consigne dans un espace de travail en réponse à des signaux provenant du capteur de pression.
